# EUROPÄISCHE PATENTSCHRIFT

(11) **EP 2 283 144 B1**
(45) Veröffentlichungstag und Bekanntmachung des Hinweises auf die Patenterteilung: **08.11.2017**
(21) Anmeldenummer: 09761586.8
(22) Anmeldetag: 22.05.2009
(51) Int. Cl.: C12P 13/02, C07H 15/00, A61K 8/68, A61Q 19/00

(54) **VERFAHREN ZUR HERSTELLUNG VON SPHINGOLIPIDEN DURCH N-ACYLIERUNG VON LYSOSPHINGOLIPIDEN UNTER VERWENDUNG EINER LIPASE UND EINES FETTSÄUREGLYCERINESTERS**
PROCESS FOR PRODUCING SPHINGOLIPIDS BY N-ACYLATION OF LYSOSPHINGOLIPIDS EMPLOYING A LIPASE AND A FATTY ACID GLYCEROL ESTER
PROCÉDÉ DE PRODUCTION DE SPHINGOLIPIDES PAR N-ACYLATION DE LYSOSPHINGOLIPIDS EN UTILISANT UNE LIPASE ET UN ESTER DE GLYCÉROL D'ACIDE GRAS

(30) Priorität: 13.06.2008 DE 102008002410
(43) Veröffentlichungstag der Anmeldung: 16.02.2011
(73) Patentinhaber: Evonik Degussa GmbH, 45128 Essen (DE)
(72) Erfinder: HOLLMANN, Frank, NL-2522 JJ Den Haag (NL); THUM, Oliver, 40880 Ratingen (DE); GRZEBYK, Pawel, 46147 Oberhausen (DE); TÖLLE, Christoph, 47167 Duisburg (DE)
(86) Internationale Anmeldenummer: PCT/EP2009/056208
(87) Internationale Veröffentlichungsnummer: WO 2009/150023

(56) Entgegenhaltungen:
- WO-A-94/26919
- WO-A-97/09307
- WO-A-2006/002909
- TAKANAMI, T. ET AL.: "Chemo-enzymatic short-step total synthesis of symbioramide" TETRAHEDRON LETTERS, Bd. 46, Nr. 19, 9. Mai 2005 (2005-05-09), Seiten 3291-3295, XP025385668
- BISTLINE JR., R.G. ET AL.: "Lipase Catalyzed Formation of Fatty Amides", JOURNAL OF THE AMERICAN OIL CHEMISTS' SOCIETY (JAOCS), vol. 68, no. 2, February 1991 (1991-02), pages 95-98, XP001180608, DOI: 10.1007/BF02662325

## Beschreibung

### Gebiet der Erfindung

Gegenstand der Erfindung ist die enzymatische Synthese von Sphingolipiden aus Lysosphingolipiden und Carbonsäureestern.

### Stand der Technik

Die Erfindung betrifft ein Verfahren zur biokatalytischen Herstellung von Sphingolipiden der allgemeinen Formel I 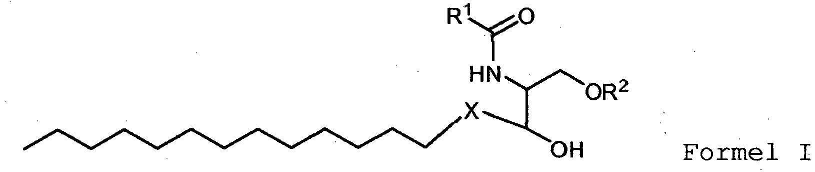 durch Umsetzung eines Lysosphingölipids der allgemeinen Formel II 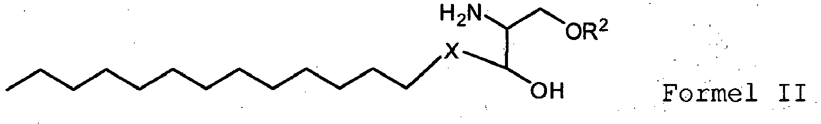 mit mindestens einem Carbonsäureester ausgewählt aus der Gruppe umfassend Verbindungen der allgemeinen Formeln IIIa-e 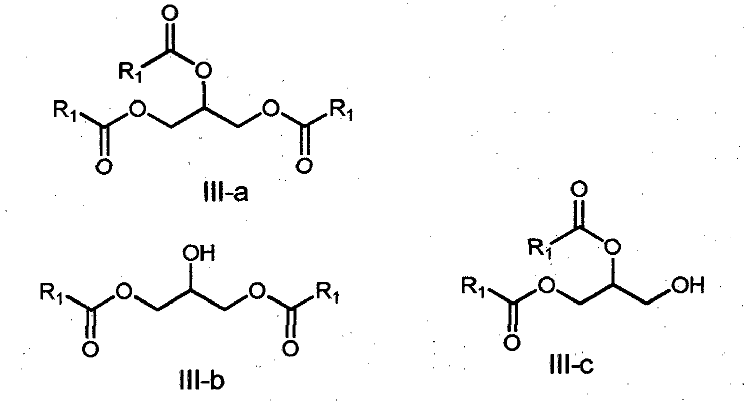 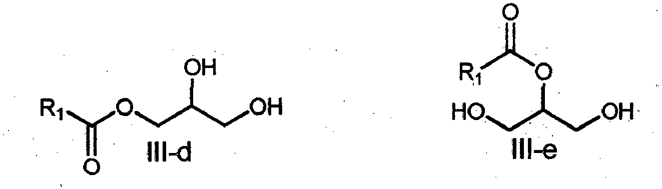 in Gegenwart eines Biokatalysators, der mindestens eine Carboxylester-Hydrolase der Enzymklasse E.C. 3.1.1 umfasst,
wobei R¹ unabhängig voneinander und gegebenenfalls unterschiedliche, lineare oder verzweigte, gegebenenfalls eine oder mehrere Mehrfachbindungen und/oder aromatische oder heteroaromatische Ringe enthaltende, gegebenenfalls durch Sauerstoffatome oder Ester- oder Amidfunktionalitäten unterbrochene, gegebenenfalls durch mindestens eine weitere Gruppe ausgewählt aus Alkyl- Hydroxy-, Keto- oder Amingruppen substituierte Alkylketten mit 2 bis 55 Kohlenstoffatomen darstellt,
R²H, Phosphocholin, Ethanolamin, Serin oder einen Zucker darstellt,
X CH=CH, CH₂ -CH₂ oder CH₂-HCOH darstellt.

Die Sphingolipide finden Anwendung als kosmetisch und/oder dermatologisch aktive Komponenten.

Sphingolipide der allgemeinen Formel I mit R² = H werden Ceramide genannt und sind natürlich in der Haut (Stratum corneum) vorkommende polare Lipide. In den äußeren Hörnzellen (Corneocyten) stellen Ceramide einen Hauptbestandteil (40-65% des Lipidanteils) der Zellmembranen dar und spielen somit eine zentrale Rolle in deren Schutzfunktion indem sie beispielsweise die Wasserpermeabilität regulieren. Mit zunehmendem Alter büßen die Keratinozyten einen Großteil ihrer Ceramidsynthese-aktivität ein wodurch die Haut einen Teil ihrer Schutzwirkung verliert und beispielsweise den epidermalen Wasserverlust nicht mehr vollständig unterbinden kann. Dies kann durch topische Applikation von Ceramiden wenigstens teilweise kompensiert werden (z.B.: Farwick, M. et al., Int. J. Cosm. Sci., 2007, 29(4), 319-329 oder Klee, S.K. et al. Basic Clinic. Dermatol., 2007, 40, 155-165).

Darüber hinaus wurden positive Effekte von Ceramiden bei der Behandlung atopischer Dermatitis berichtet (Kerscher, M. et al., Eur. J. Dermatol., 1991, 1, 39-43; Imokawa, G. et al., J. Soc. Cosmet. Chem. 1989, 40, 500-507).

In Anbetracht der demographischen Entwicklung in vielen Industrieländern, insbesondere in Deutschland, ist mit einem weiter wachsenden Bedarf an Ceramiden zu rechnen.

Nach dem Stand der Technik werden Ceramide durch Schotten-Baumann-analoge N-Acylierung von Lysosphingolipiden der allgemeinen Formel II mit R² = H wie z.B. Phytosphingosin (allgemeine Formel II mit R² H und X = CH₂-HCOH) mittels aktivierter Carbonsäurederivate hergestellt.

Typischerweise ist das Phytosphingosin fermentativen Ursprungs.

Bei der aktivierten Carbonsäure handelt es sich typischerweise um das Carbonsäurechlorid welches entweder als solches eingesetzt wird oder in *situ* aus der Carbonsäure hergestellt wird. In US6420604 (Cosmoferm, NL) wird die Synthese einiger Ceramide durch Reaktion der Sphingosinbase mit entsprechenden Carbonsäurehalogeniden beschrieben. Besonders nachteilig bei dieser Methode ist einerseits die Notwendigkeit des Einsatzes von toxischen Organochlorverbindungen, sowie das Anfallen einer hohen Salzfracht im Endprodukt. Außerdem ist dem Fachmann offensichtlich, dass bei der Verwendung hochreaktiver Carbonsäurehalogenide mit der Bildung signifikante Mengen unerwünschter Nebenprodukte zu rechnen ist.

Alternative Syntheserouten verlaufen über Anhydride. So lehrt beispielsweise WO93/20038 (Gist-Brocades, NL) die basenkatalysierte Synthese gemischter Anhydride aus Carbonsäure und Alkyl-phenylsulfonylchlorid um reaktive Carbonsäurederivate für die N-Acylierung von Phytosphingosin zu erhalten.

Allen diesen Routen ist gemein, dass sie auf der Verwendung reaktiver Carbonsäurederivate beruhen. Nachteilig ist hierbei sowohl die hohe Korrosivität dieser Substanzen als auch ihre Gesundheitsschädlichkeit was spezielle Reaktorsysteme und Vorkehrungen notwendig macht und somit den präparativen Aufwand erhöht. Darüber hinaus muss für die topische Anwendbarkeit der Produkte sichergestellt sein dass sie von den reaktiven und toxischen Edukten und Nebenprodukten befreit sind. Des Weiteren ist mit hohen Salzfrachten zu rechnen sowie dem damit verbundenen zusätzlichen Aufwand bei der Produktaufreinigung und Entsorgung. Ein weiterer Nachteil der Verfahren des Standes der Technik ist, dass nur geringe Edukt- und Produktkonzentrationen erhalten werden; so sind in WO93/20038 (Gist-Brocades, NL) maximal 15% (w/v)-Eduktlösungen im toxischen Lösungsmittel Methylenchlorid bei gleichzeitiger Verwendung von reaktiven und toxischen Coreagentien p-Toluolsulfonsäurechlorid und Triethylamin beschrieben. Darüber hinaus ist sind entweder Reinheit der Produkte oder Ausbeuten (jeweils im Bereich um 80%) nicht zufriedenstellend.

Ein weiterer Nachteil ist die Beschränkung auf isolierte Fettsäuren, bzw. Fettsäurederivate. Natürlich kommen Fettsäuren hauptsächlich als Glyceride vor (Fette und Öle) aus denen die Fettsäuren erst gewonnen werden müssen. Vor allem bei sehr empfindlichen Fettsäuren, wie z. B. mehrfach ungesättigten Fettsäuren, sind hier aufwändige Verfahren notwendig, um die Fettsäuren unter Vermeidung von Nebenreaktionen zu erhalten. Bisher ist kein Verfahren zur Herstellung von Ceramiden ausgehend von natürlich vorkommenden Glyceriden wie z.B. Olivenöl, Sonnenblumenöl, Leinöl, Rapsöl, Distelöl, Sojaöl, Maiskeimöl, Erdnussöl, Palmöl, Palmkernöl, Kokosfett, Rizinusöl, Sesamöl, Avocadoöl, Schweineschmalz, Rindertalg, Heringsöl, Kamelienöl, Kakaobutter, Kürbiskernöl, Mandelöl, Mangobutter, Nachtkerzenöl, Pistazienöl, Pekannussöl, Reisöl, Sanddornöl, Senföl, Schwarzkümmelöl, Sheabutter, Walnussöl, Tungöl, Traubenkernöl bekannt.

Aufgabe der vorliegenden Erfindung war deshalb die Bereitstellung eines alternativen, schonenden und industriell anwendbaren Zugangs zu Ceramiden bei dem wenigstens einer der Nachteile des Standes der Technik überwunden werden kann.

Amidierungsreaktionen sind auch biokatalytisch, d.h. unter Verwendung eines Enzyms als Katalysator, zugänglich. Übersichten über industrielle Anwendungen von Enzymen finden sich beispielsweise bei Liese et al (Industrial Biotransformations; Second, Completely Revised Edition, Wiley-VCH, Weinheim, 2006). Bevorzugte Biokatalysatoren für die Synthese von Carbonsäurederivaten sind Hydrolasen (E.C. 3.1.x.x) insbesondere Lipasen (Triacylglycerinesterhydrolasen, E.C. 3.1.1.3) und Esterasen (E.C. 3.1.1.1).

Entsprechend ihrer natürlichen Funktion im Metabolismus einer Zelle katalysieren Lipasen bevorzugt hydrolytische Spaltung von Estern. Aber auch die kondensative Bildung von Estern ist vielfach literaturbeschrieben. Repräsentative Beispiele hierfür finden sich bei Drauz und Waldmann (Enzyme Catalysis in Organic Synthesis, A Comprehensive Handbook; Second, Completely Revised and Enlaged Edition, Vol. II, Wiley-VCH, Weinheim, 2002), bei Aehle (Enzymes in Industry, Production and Applications; Second, Completely Revised Edition, Wiley-VCH, Weinheim, 2004) oder bei Bornscheuer (Enzymes in Lipid Modification, Wiley-VCH, Weinheim, 2000).

Auch für die Verwendung von Aminen als Nucleophile in Lipase-katalysierten Kondensationsreaktionen finden sich Literaturbeispiele.

Y.-M. Xia et al, J Mol Catal B, 2004, 31, 111-115 beschreiben beispielsweise die Synthese von N-Lauroyl-β-aminoproprionitril durch Amidierung von Laurinsäuremethylester mit einem reaktiven, primären Amin katalysiert durch eine Lipase aus *Candida antarctica* (CALB). Nachteilig bei dem beschriebenen Verfahren ist neben der Beschränkung auf verdünnte Substratlösungen (unter 50 mM) der vergleichweise geringe Umsatz von lediglich bis zu 94,3% Umsatz. Darüber hinaus ist bei der gezeigten Reaktion keinerlei Regio- oder Chemoselektivität notwendig.

Solche Selektivitäten können allerdings bei der erfindungsgemäßen Umsetzung zwingend notwendig, da sich in einem Eduktmolekül wie beispielsweise dem Phytosphingosin mehrere reaktive Funktionalitäten befinden können. Die Problematik dieser Selektivität zeigt sich beispielsweise bei P. Tufvesson et al.: Biotechnol.Bioeng., 2007, 97(3), 447-453: Bei der CALB-katalysierten Umsetzung von Ethanolamin mit Carbonsäuren war keine einfache selektive Amidierung unter Umgehung der enzymkatalysierten Veresterung möglich. Lediglich unter mehrfachem Zudosieren eines Eduktes und destillativer Entfernung des Reaktionswassers konnte eine Anreicherung des gewünschten Amids erreicht werden.

In einem weiteren Beispiel für die enzymatische Amidierung beschreiben M. Nechab et al.: JOC, 2007, 72, 6918-6923 die stereoselektive Umsetzung von (R)-konfigurierten sekundären Aminen mittels CALB. Die hohe optische Reinheit (>99% ee) der beobachteten Produkte legt eine starke Präferenz der CALB für (R)-konfigurierte Amine nahe, wodurch die Umsetzung von beispielsweise Phytosphingosin aufgrund seiner S-Konfiguration am Amin-Kohlenstoffatom mit diesem Katalysator fraglich ist. Des Weiteren wurden wiederum stark verdünnte Substratlösungen (100 mM) bei gleichzeitig hohen Biokatalysatorkonzentrationen (27% w(CALB)/w(Substrate)) eingesetzt was die industrielle Attraktivität des beschriebenen Verfahrens signifikant einschränkt.

Die Patentschrift WO94/26919 (Gist-Brocades, NL) offenbart ein Verfahren zur biokatalytischen Herstellung eines Sphingolipids der Formel I durch Umsetzung des entsprechenden Lysosphingolipids der Formel II mit dem Carbonsäureester Stearinsäuremethylester in Gegenwart einer Lipase.

Das Dokument TAKANAMI, T. ET AL.: "Chemo-enzymatic short-step total synthesis of symbioramide": TETRAHEDRON LETTERS, 2005, 46(19), 3291-3295 offenbart ein Verfahren zur biokatalytischen Herstellung eines Sphingolipids der Formel I, nämlich Symbioramid, durch Umsetzung des entsprechenden Lysosphingolipids der Formel II mit dem Carbonsäureester Methyl(2R,3E)-2-hydroxy-3-octadecenoat in Gegenwart einer Lipase.

Das Dokument BISTLINE, R.G. ET AL.: "Lipase Catalyzed Formation of Fatty Amides": JOURNAL OF THE AMERICAN OIL CHEMISTS' SOCIETY (JAOCS), 1991, 68(2), 95-98 offenbart ein Verfahren biokatalytischen Herstellung eines Fettsäureamids durch Umsetzung des entsprechenden Alkylamins mit einem Carbonsäureester der Formel IIIa in Gegenwart einer Lipase.

In WO94/26919 ist die enzymatische N-Acylierung von Phytosphingosin mittels Carbonsäureestern und unter Verwendung bakterieller Lipasen oder Säugetierlipasen beschrieben. In dem beschriebenen Verfahren werden bevorzugt bakterielle Lipasen und Säugetierlipasen eingesetzt; insbesondere eine Lipase aus *Pseudomonas alcaligenes.* Nachteilig bei diesem Verfahren ist insbesondere die Notwendigkeit hoher Biokatalysatormengen (30-95% w/w) zur Erreichung von nur mäßigen Umsätzen (bis zu 78%). Darüber hinaus wurden signifikante Mengen an unerwünschten N,O-Di-acylierungsprodukten (bis zu 17%) gefunden. Zusätzlich wurden lediglich verdünnte Lösungen der Substrate eingesetzt (59 bis 93 mM) was geringe Raum-Zeit-Ausbeuten zur Folge hatte. Ein weiterer Nachteil ist die Beschränkung auf Phytosphingosin-Base als Substrat was im Vergleich zur Verwendung von Säureadditionsprodukten des Phythosphingosins, beispielsweise Phytosphingosin-Sulfat, eine drastische Erhöhung der Substratkosten mit sich bringt. Ausdrücklich wird außerdem darauf hingewiesen, dass Lipasen aus Hefen und Pilzen als Katalysatoren nicht geeignet sind. Somit scheidet das in WO94/26919 beschriebene Verfahren als ökonomisch sinnvolle Alternative zu den bestehenden chemischen Verfahren aus.

Es besteht daher weiterhin Bedarf an Methoden der enzymatischen Amidierung von Lysosphingolipiden, welche die Nachteile des Standes der Technik überwinden.

### Beschreibung der Erfindung

Aufgabe der vorliegenden Erfindung war daher die Bereitstellung eines Verfahrens, das einen oder mehrere Nachteile der Verfahren des Standes der Technik nicht aufweist. Insbesondere sollte ein Verfahren entwickelt werden, mit dem gut verfügbare Edukte und darüber hinaus diese in hohen Konzentrationen eingesetzt werden können.

Weitere nicht explizit genannte Aufgaben ergeben sich aus dem Kontext der nachfolgenden Beschreibung, der Beispiele sowie der Ansprüche.
Völlig überraschend wird die Aufgabe der vorliegenden Erfindung durch den Einsatz von Voll- oder Partialestern des mehrwertigen Alkohols Glycerin als Acyldonoren für die Darstellung von Sphingolipiden gelöst.

Diese Form der Acyldonoren, z.B. als Fette und Öle, also von Estern des dreiwertigen Alkohols Glycerin, ist bisher nicht beschrieben worden.

Dies liegt vor allen Dingen daran, dass der Fachmann erwarten würde, dass sich bei Verwendung von z.B. Fetten und Ölen im Verlauf der Reaktion Mono- und Diglyceride in signifikanten Mengen ansammeln. Dadurch wäre die gewünschte Umsetzung nicht leicht möglich, insbesondere hätten die zu erwartenden komplexen und undefinierten Reaktionsmischungen zu Produkten mit nicht befriedigenden Reinheiten geführt, wodurch deren Anwendung in kosmetischen und/oder dermatologischen Anwendungen nicht möglich ist.

Darüber hinaus weisen Mono- und Diacylglyceride einen erheblichen tensidischen Charakter auf, welcher die Aufarbeitung der Reaktionsmischung deutlich erschwert. Zudem können tensidische Moleküle die Aktivität und Stabilität von Enzymen empfindlich beeinträchtigen, indem sie beilspielsweise den Biokatalysator von seinem Trägermaterial ablösen (*leaching*)*.* Dies wäre aus zweierlei Hinsicht fatal für einen angestrebten biokatalytischen Prozess: einerseits würde durch *leaching* der Katalysator verbraucht was häufiges Nachdosieren frischen Katalysators bedeutete und somit die ökonomische Attraktivität der Verfahrens beeinträchtigen würde. Andererseits würde durch *leaching* das Produkt mit Enzym kontaminiert, wodurch dessen Anwendung in kosmetischen und/oder dermatologischen Formulierungen insbesondere aufgrund der Gefahr von allergischen Reaktionen auf das Enzym unmöglich würde.

Überraschenderweise wurde gefunden, dass sich die erwähnten Mono- und Diglyceride zu keinem Zeitpunkt des erfindungsgemäßen Verfahrens in nennenswerten Mengen nachweisen lassen. Als einziges Nebenprodukt konnte bei Einsatz von Fetten/Ölen Glycerin nach Beendigung der Reaktion nachgewiesen werden.

Ein Vorteil des erfindungsgemäßen Verfahrens ist daher die Vermeidung oben genannter Nachteile der beispielhaften Mono- oder Diglyceride.

Ein weiterer Vorteil des erfindungsgemäßen Verfahrens ist, dass der reine mehrwertige Alkohol Glycerin ein kosmetisch und dermatologisch weit verbreitetes Lösungsmittel darstellt, welches auch in hohen Mengen im Produkt verbleiben kann. Alternativ kann Glycerin leicht durch einen Extraktionsschritt mit Wasser aus dem Endprodukt entfernt werden.

Noch ein weiterer Vorteil des erfindungsgemäßen Verfahrens ist, dass dieses durch den Einsatz von Fetten und Ölen, besonders von natürlichen Fetten und Ölen, gut zugängliche Edukte verbraucht und somit aus ökonomischer Sicht einen deutlichen Vorteil im Vergleich zum Einsatz schwer zugänglicher (teil-)synthetischer Methyl-, Ethyl- und Propylester darstellt.

Das erfindungsgemäße Verfahren hat weiterhin den Vorteil, dass Amidierungsreaktion ökonomisch sinnvoll durchgeführt werden können, da entweder relativ niedrige Enzymkonzentrationen eingesetzt werden müssen, beispielsweise unter 10 Gewichts-% bezogen auf die eingesetzten Edukte, und/oder der Biokatalysator mehrfach, zum Beispiel mindestens 10-fach, wieder verwendet werden kann. Ein weiterer Vorteil des erfindungsgemäßen Verfahrens besteht darin, dass ohne aufwändige Aufarbeitungsverfahren, wie Chromatographie oder fraktionierte Kristallisation Produkte hoher Reinheit, beispielsweise mit einem Aktivgehalt von > 90% und einem Anteil von N,O-Diacylierungsprodukten von unter 5% darstellbar sind.

Gegenstand der vorliegenden Erfindung ist daher ein Verfahren zur biokatalytischen Herstellung von Sphingolipiden der allgemeinen Formel I 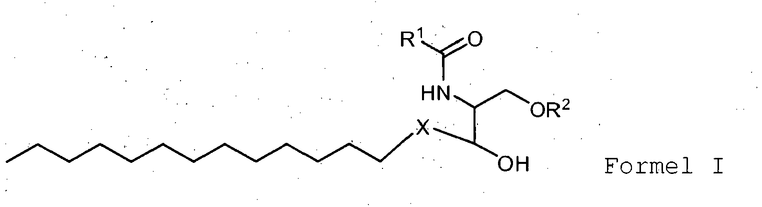 durch Umsetzung eines Lysosphingolipids der allgemeinen Formel II 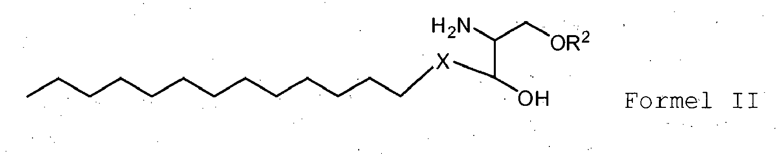 mit mindestens einem Carbonsäureester ausgewählt aus der Gruppe umfassend Verbindungen der allgemeinen Formeln IIIa-e 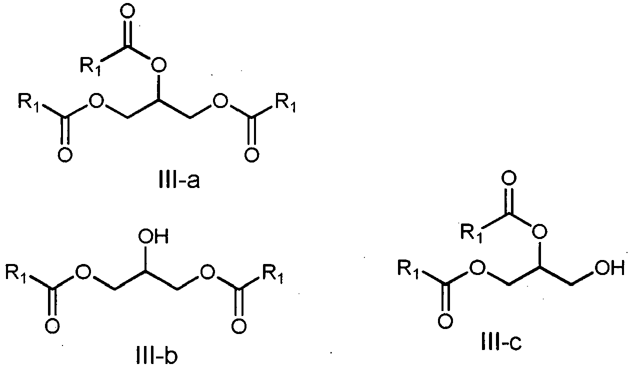 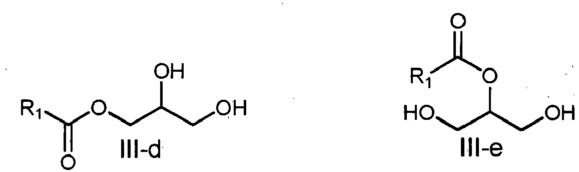 in Gegenwart eines Biokatalysators, der mindestens eine Carboxylester-Hydrolase der Enzymklasse E.C. 3.1.1 umfasst,
wobei R¹ unabhängig voneinander und gegebenenfalls unterschiedliche, lineare oder verzweigte, gegebenenfalls eine oder mehrere Mehrfachbindungen und/oder aromatische oder heteroaromatische Ringe enthaltende, gegebenenfalls durch Sauerstoffatome oder Ester- oder Amidfunktionalitäten unterbrochene, gegebenenfalls durch mindestens eine weitere Gruppe ausgewählt aus Alkyl- Hydroxy-, Keto- oder Amingruppen substituierte Alkylketten mit 2 bis 55 Kohlenstoffatomen darstellt,
R² H, Phosphocholin, Ethanolamin, Serin oder einen Zucker darstellt,
X CH=CH, CH₂-CH₂ oder CH₂-HCOH darstellt.

Bevorzugt ist die Carboxylester-Hydrolase der Enzymklasse E.C. 3.1.1 eine, die sich aus einem Organismus des Reiches der Pilze isolieren lässt und Carboxylester-Hydrolasen der Enzymklasse E.C. 3.1.1, die auf Aminosäureebene mindestens 60 %, vorzugsweise mindestens 80 % bevorzugt mindestens 90 %, besonders bevorzugt mindestens 95%, 98% bzw. 99 % homolog zu den sich aus einem Organismus des Reiches der Pilze isolieren lassenden sind.

Mit "Homologie auf Aminosäureebene" im Sinne der vorliegenden Erfindung ist nun und sei auch im Folgenden die "Aminosäure-Identität" verstanden, welche mit Hilfe bekannter Verfahren bestimmt werden kann. Generell werden besondere Computerprogramme mit Algorithmen unter Berücksichtigung spezieller Erfordernisse verwendet. Bevorzugte Verfahren zur Bestimmung der Identität erzeugen zunächst die größte Übereinstimmung zwischen den zu vergleichenden Sequenzen. Computer-Programme zur Bestimmung der Identität umfassen, sind jedoch nicht beschränkt auf, das GCG- Programmpaket, einschließlich
- GAP (Deveroy, J. et al., Nucleic Acid Research 12 (1984), Seite 387, Genetics Computer Group University of Wisconsin, Medicine (WI), und
- BLASTP, BLASTN und FASTA (Altschul, S. et al., Journal of Molecular Biology 215 (1990), Seiten 403-410. Das BLAST-Programm kann erhalten werden vom National Center For Biotechnology Information (NCBI) und aus weiteren Quellen (BLAST Handbuch, Altschul S. et al., NCBI NLM NIH Bethesda ND 22894; Altschul S. et al., vorstehend).

Der Fachmann ist sich bewusst, dass verschiedene Computerprogramme für die Kalkulation der Ähnlichkeit bzw. Identität zwischen zwei Nukleotid- oder Aminosäure-Sequenzen zur Verfügung stehen. So kann die prozentuale Identität zwischen zwei Aminosäure-Sequenzen z.B. durch den Needleman und Wunsch (J. Mol. Biol. (48): 444-453 (1970)) Algorithmus bestimmt werden, der in das GAP Programm im GCG Software-Paket (erhältlich über http://www.gcg.com) integriert wurde, und zwar entweder unter Verwendung einer Blossom 62-Matrix oder einer PAM250-Matrix, einer gap weight von 16, 14, 12, 10, 8, 6, oder 4 und einer length weight von 1, 2, 3, 4, 5, oder 6. Der Fachmann wird anerkennen, dass die Verwendung unterschiedlicher Parameter zu leicht unterschiedlichen Ergebnissen führen wird, dass aber die prozentuale Identität zwischen zwei Aminosäure-Sequenzen insgesamt nicht signifikant unterschiedlich sein wird. Üblicherweise wird die Blossom 62-Matrix unter Anwendung der Voreinstellungen (gap weight: 12, length weight: 1) genutzt.

Eine Identität von 60 % gemäß dem vorstehenden Algorithmus bedeutet im Zusammenhang mit der vorliegenden Erfindung 60 % Homologie. Das gleiche gilt für höhere Identitäten.

Besonders bevorzugt ist in dem erfindungsgemäßen Verfahren der Carbonsäureester ausgewählt aus der Gruppe umfassend Verbindungen der allgemeinen Formeln IIIa-e der Ester mindestens einer Carbonsäure mit Glycerin, bei dem die Carbonsäure ausgewählt ist aus der Gruppe der natürlich vorkommenden Fettsäuren auf Basis natürlicher pflanzlicher oder tierischer Öle mit 6-30 Kohlenstoffatomen, insbesondere mit 8-22 Kohlenstoffatomen. Natürliche Fettsäuren sind unverzweigt und bestehen aus einer geraden Anzahl Kohlenstoffatomen. Eventuelle Doppelbindungen besitzen cis-Konfiguration. Beispiele sind: Capronsäure, Caprylsäure, Caprinsäure, Laurinsäure, Myristinsäure, Palmitinsäure, Palmitoleinsäure, Isostearinsäure, Ste arinsäure, 12-Hydroxystearinsäure, Dihydroxystearinsäure, Ölsäure, Linolsäure, Linolensäure, Petroselinsäure, Elaidinsäure, Arachinsäure, Behensäure, Erucasäure, Gadoleinsäure, Eicosapentaensäure, Docosahexaensäure, Arachidonsäure, 9- oder 13-Hydroxyoctadecadiensäure, 15-Hydroxyeicosatetraensäure und die Reihe der sogenannten alpha-Hydroxysäuren. Ebenfalls ist in diesem Zusammenhang die Carbonsäure besonders bevorzugt ein Polykondensationsprodukt von hydroxyfunktionalisierten Säuren, beispielsweise Poly-12-hydroxystearinsäure oder Polyricinolsäure.

Erfindungsgemäß können auch die Säureadditionsprodukte, auch Säureadditionsalze genannt, der Lysosphingolipide eingesetzt werden, wie sie beispielsweise im Falle des bereits genannten Phytosphingosins bei den etablierten fermentativen Verfahren anfallen, wie beispielsweise beschrieben bei P. Lersch, U. Schick, Spec. Chem. Mag., 2003, 23(6), 30-31. Als Säureadditionsprodukt des Lysosphingolipids wird bevorzugt das Carbonsäurecarboxylat, Sulfat, Phosphat, Nitrat, Carbonat, Hydroxid oder Halogenid, besonderd bevorzugt das Chlorid und Sulfat des Lysosphingolipids eingesetzt.

Bei Verwendung des Säureadditionsproduktes des Lysosphingolipids in erfindungsgemäßem Verfahren ist es bevorzugt, dass das Lysosphingolipid durch Deprotonierung des Säureadditionsprodukts des Lysosphingolipids vor der enzymatischen Umsetzung hergestellt wird.

Das so erhaltene Lysosphingolipid wird im Folgenden auch als Lysosphingolipid-Base bezeichnet.

Die deprotonierende Behandlung kann in Lösungen oder Suspensionen des Säureadditionsproduktes des Lysosphingolipids in üblichen organischen Lösungsmitteln stattfinden. Als Lösungsmittel können beispielsweise eingesetzt werden: Paraffine, ein- oder mehrwertige Alkohole (z.B.: Methanol, Ethanol, Isopropanol, Propanol, Butanol, Pentanol, Hexanol, Cyclohexanol, Methylcyclohexanol, 2-Butyl-1-octanol sowie deren Isomere, Ethylenglycol, Glycerol, Diacetonalkohol, Isobutanol, etc.), Ether (Diethylether, tert.-Butylmethylether, Tetrahydrofuran, Dioxan, Polyethoxylate, Polypropoxylate sowie Mischpolymere etc.), Ketone (Azeton, Methylisobutylketon, Methylethylketon, etc.), Ester (Triethylcitrat, Tributylcitrat. Isobutylisobutyrat, Isobutylacetat. Isononylisononanoat,(2-Ethylhexyl)Acetat, Cyclohexylacetat, 4-tert.-Butylcyclohexylacetat). Bevorzugt sind toxikologisch unbedenkliche Lösungsmittel wie Methylisobutylketon oder 2-Methyl-2-butanol.

Der Deprotonierungsschritt kann bei Reaktionstemperaturen durchgeführt werden, bei denen das Lösungsmittel sich im flüssigen Zustand befindet. Vorzugsweise bei Reaktionstemperaturen zwischen -80°C und +150°C, besonders bevorzugt zwischen 0°C und 120°C, ganz besonders bevorzugt zwischen +25°C und 100°C.

Die so erhaltene Lösung von Lysosphingolipid-Base kann als solche für die enzymatische Umsetzung eingesetzt werden oder vorher filtriert werden.

In einer weiteren Ausführung der vorliegenden Erfindung kann das Lösungsmittel des Deprotonierungsschritts nach einer dem Fachmann geläufigen Methode (z.B. destillative Entfernung oder Präzipitation/Kristallisation der Lysosphingolipid-Base mit anschließender Filtration) entfernt werden. Optional kann die Lysosphingolipid-Base vor der enzymatischen Umsetzung beispielsweise durch Präzipitation oder Kristallisation oder durch destillative Entfernung des Lösungsmittels isoliert werden. Bevorzugt ist, dass zwischen der Deprotonierung des Säureadditionsprodukts des Lysosphingolipids und biokatalytischer Herstellung ein Filtrationsschritt erfolgt, in dem die bei der deprotonierenden Behandlung anfallenden Salze entfernt werden.

Alternativ kann die Lysosphingolipid-Base durch Deprotonierung des Säureadditionsprodukts des Lysosphingolipids während der Biokatalyse hergestellt werden.

Die mit einer Aktivierung einhergehende Deprotonierung kann durch Einsatz einer Base erfolgen, bevorzugt mittels einer organischen oder anorganischen Base. Bevorzugt werden als anorganische Basen anorganische Hydroxide, Carbonate, Metallhydride (wie z.B.: Lithiumaluminiumhydrid, Calziumhydrid, Natriumhydrid und ähnliche), Ionenaustauschermaterialien (wie z.B. Kationen- oder Anionentauscher) sowie als organische Basen Metallorganyle (wie beispielsweise Butyllithium) Alkoholate, Amide sowie deren Metallsalze wie beispielsweise Lithiumdiisopropylamin eingesetzt.

Überraschenderweise wurde gefunden, dass insbesondere solche Basen, bei deren Reaktion mit den Säureadditionssalzen formal kein Wasser entsteht, qualitative besonders hochwertige Lysoshingolipid-Base liefen, welche sich in der enzymatischen Reaktion besonders leicht umsetzen lässt. Besonders bevorzugt sind daher solche Basen, bei denen kein Reaktionswasser frei wird. Dies sind solche, die nach Protonierung kein Wasser frei setzen. Bevorzugt eingesetzt wird als Base ein Alkalimetallalkoholat. Dieses kann beispielsweise in alkoholischer Lösung vorliegen. Besonders bevorzugt sind Natrium- und Kaliummethanolat. Diese können ebenfalls als Lösungen in organischen Lösungsmitteln eingesetzt werden.

Eine weitere Alternative, Reaktionswasser zu vermeiden, und somit ebenso bevorzugt in erfindungsgemäßem Verfahren ist der Einsatz von wasserbindenden Salzen, bevorzugt wasserbindenden anorganische Salzen, wie beispielsweise Na₂SO₄ zur Bindung von entstehendem Reaktionswasser bei Einsatz von Alkali- und/oder Erdalkalihydroxiden als Base. Bevorzugt wird Na₂SO₄ eingesetzt.

Erfindungsgemäß liegt in dem Verfahren das molare Verhältnis zwischen Säureadditionsprodukts des Lysosphingolipids und Base im Bereich zwischen 10 zu 1 bis 0,05 zu 1, bevorzugt zwischen 3 zu 1 bis 0,2 zu 1, besonders bevorzugt zwischen 1,4 zu 1 und 0,6 zu 1 und ganz besonders bevorzugt ist das molare Verhältnis zwischen Säureadditionsprodukt des Lysosphingolipids und Base äquimolar.

Bevorzugt ist in dem erfindungsgemäßen Verfahren der Gehalt an entstehenden Partialglyceriden kleiner als 2,5% w/w, besonders bevorzugt kleiner als 1% w/w, ganz besonders bevorzugt kleiner als 0,5% w/w bezogen auf die Masse der Einwaage des Carbonsäureesters der allgemeinen Formeln IIIa-e als Edukt,

In einer besonderen Ausführung der vorliegenden Erfindung wird eventuell vorhandenes Wasser aus dem Deprotonierungs ansatz zur Herstellung der Lysosphingolipid-Base entfernt. Beispielsweise werden hierzu Wasserentfernungsverfahren wie physikalischer Methoden (z.B. Destillation, Membranverfahren, Extraktion oder Adsorption (beispielweise an Molekularsieb)) oder auch chemische Methoden (z.B.: Reaktion mit Metallhydriden und -Oxiden oder anderen Reaktanden wie Anhydriden, Estern) eingesetzt.

Wie bereits eingangs erwähnt, ist es vor dem Stand der Technik, wie er in WO94/26919 (Gist-Brocades) beschrieben ist, völlig überraschend, dass Pilzlipasen in erfindungsgemäßem Verfahren als effizienter Biokatalysator eingesetzt werden können.

In dem erfindungsgemäßen Verfahren werden als Biokatalysator insbesondere Carboxylester-Hydrolasen der Enzymklasse E.C. 3.1.1, die sich aus einem Organismus des Reiches der Pilze isolieren lassen und/oder die auf Aminosäureebene mindestens 60 %, vorzugsweise mindestens 80 % bevorzugt mindestens 90 %, besonders bevorzugt mindestens 95%, 98% bzw. 99 % homolog zu den sich aus einem Organismus des Reiches der Pilze isolieren lassenden sind, bei denen der Organismus ausgewählt ist aus der Gruppe der Gattungen *Aspergillus, Bipolaris*, *Candida, Fusarium*, *Geotrichum, Humicola, Microsporum, Mucor, Penicillium, Pichia, Rhizopus, Rhizomucor, Nocardia, Saccharomyces, Streptomyces, Thermomyces, Trichosporon, Zygosaccharomyces.*

Beispielhafte Vertreter sind z.B.: *Aspergillus caesiellus, A. candidus, A. carbonarius, A. carneus, A. chevalieri, A. clavatus, A. costaricaensis, A. cretensis, A. deflectus, A. flaviceps, A. flavus, A. flocculatosus, A. fumigatus, A. glaucus, A. ibericus, A. lacticoffeatus, A. lentulus, A*. *neobridgeri, A. nidulans, A. niger, A. niveus, A. ochraceus, A. oryzae, A. parasiticus, A. penicilloides, A. piperis, A. pseudoelegans, A. pseudofisheri, A. restrictus, A. roseoglobulus, A. sclerotioniger, A. sojae, A. steynii, A. sydowi, A. terreus, A. udagawae, A. ustus, A. versicolor, A. westerdijkiae, Bipolaris australiensis, B.hawaiiensis, B. picifera, Candida antarctica, C. cylindracea, C. lipolytica, C. rugosa, C. utilis, C. robusta, C. kefyr, C. albicans, C. glabrata, C. krusei, C. lusitaniae, C. parapsilosis, C. tropicalis, Fusarium chlamydosporum, F. coeruleum, F. dimerum, F. incarnatum, F. moniliforme F. oxysporum, F. proliferatum, F. sacchari, F. semitectum, F. solani, F. sporotrichoides, F. sub glutinans, F. tabacinum, F. verticillioides, Geotrichum candidum, G.klebahnii, Humicola fuscoatra var. Fuscoatra, H.a grisea var. Grisea, H. grisea var. Thermoidea, H. insolens, Microsporum amazonicum, M. audouinii, M. audouinii var. Langeronii, M. audouinii var. Rivalierii, M. boullardii, M. canis, M. canis var. distortum, M. cookei, M. equinum, M. ferrugineum, M. fulvum, M. gallinae, M. gypseum, M. nanum, M. persicolor, M. praecox, M. racemosum, M. vanbreuseghemii Mucor javanicus, M. pusillus, M. plumbeus, M. racemosus, M. hiemalis, Nocardia asteroides, N. brasiliensis, N. otitidiscaviarum, Penicillium aurantiogriseum, P. brevicompactum, P. chrysogenum, P. camemberti, P. digitatum, P. citrinum, P. commune, P. corylophilum, P. crustosum, P. cyclopium, P. expansum, P. funiculosum, P. glabrum, P. glaucum, P. griseofulvum, P. italicum, P. marneffei, P. nalgiovense, P. nordicum, P. notatum, P. palitans, P. purpurrescens, P. purpurogenum, P. olsonii, P. oryzae, P. roqueforti, P. variabile, P. viridicatum, P. verrucosum, Pichia acaciae, P. amylophilia, P. ciferrii, P. pastoris, P. alni, P. americana, P. amethionina, P. angophorae, P. angusta, P. anomala, P. antillensis, P. bar keri, P. besseyi, P. bimundalis, P. bispora, P. bovis, P. cactophila, P. canadensis, P. capsulata, P. caribaea, P. castillae, P. chambardii, P. delftensis, P. deserticola, P. dryadoides, P. euphorbiae, P. euphorbiiphila, P. fabianii, P. faecalis, P. farinosa, P. fermentans, P. finlandica, P. fluxuum, P. galaeiformis, P. glucozyma, P. guilliermondii, P. hampshirensis, P. haplophila, P. heedii, P. heimii, P. henricii, P. holstii, P. inositovora, P. jadinii, P. japonica, P. kluyveri, P. kodamae, P. lyn ferdii, P. maganishii, P. media, P. membranifaciens, P. methanolica, P. methylivoria, P. mexicana, P. meyerae, P. minuta, P. mississippiensis, P. nakasei, P. nakazawae, P. norvegensis, P. oezlemii, P. ofunaensis, P. ohmeri, P. onychis, P. opuntiae, P. petersonii, P. philodendri, P. philogaea, P. pijperi, P. pini, P. populi, P. pseudocactophila, P. quercuum, P. rabaulensis, P. rhodanensis, P. salicaria, P. scolyti, P. segobiensis, P. silvicola, P. spartinae, P. stipitis, P. strasburgensis, P. subpelliculosa, P. sydowiorum, P. tannicola, P. thermotolerans, P. toletana, P. trehalophila, P. triangularis, P. veronae, P. wickerhamii, P. xylosa, Thermomyces lanuginosa, Rhizopus arrhizus, R. delemar, R. niveus, R. oryzae, R. azygosporus, R. microsporus, R. schipperae, R. stolonifer, Rhizomucor miehei, Saccharomyces barnettii,* S. *bayanus, S. castellii, S. cerevisiae, S. dairenensis, S. exiguus, S. paradoxus, S. pastorianus, S. rosinii, S. servazii, S. spencoerorum, S. transvaalensis, S. unisporus, S. bailii, Streptomyces spp., Trichosporon asahii, T. asteroides, T. beigelii, T. coremiiforme, T. cutaneum, T. faecale, T. gracile, T. inkin, T. mucoides, T. ovoides, T. pullulans, Zygosaccharomyces bisporus, Z. cidri, Z. fermantati, Z. florentinus, Z. mellis, Z. microellipsoides, Z. mrakii, Z. rouxii.*

Besonders bevorzugt eingesetzte Carboxylester-Hydrolasen in erfindungsgemäßem Verfahren sind Enzyme ausgewählt aus der Gruppe der Lipase aus *Thermomyces lanuginosus* (accessionnumber *059952),* die Lipasen A und B (accessionnumber P41365)aus *Candida antarctica* und, die Lipase aus *Mucor miehei* (accessionnumber *P19515),* die Lipase aus *Humicola sp.* (accessionnumber 059952), die Lipase aus *Rhizomucor javanicus* (accessionnumber S32492), die Lipase aus *Rhizopus* oryzae (accessionnumber P61872), die Lipasen aus *Candida rugosa* (accessionnumber P20261, P32946, P32947, P3294 und P32949), die Lipase aus *Rhizopus niveus* (accessionnumber *P61871),* die Lipase aus *Penicillium camemberti* (accessionnumber P25234), die Lipasen aus *Aspergillus niger* (ABG73613, ABG73614 und ABG37906) und die Lipase aus *Penicillium cyclopium* (accessionnumber P61869), so wie jeweils deren auf Aminosäureebene mindestens 60 %, vorzugsweise mindestens 80 % bevorzugt mindestens 90 %, besonders bevorzugt mindestens 95%, 98% bzw. 99 % homologen. Bezüglich Homologie sei auf oben genannte Definition verwiesen.

Kommerzielle Beispiele und ebenfalls bevorzugt eingesetzte Carboxylester-Hydrolasen in erfindungsgemäßem Verfahren sind die Handelsprodukte Lipozyme TL IM, Novozym 435, Lipozyme IM 20, Lipase SP382, Lipase SP525, Lipase SP523, (alles Handelsprodukte der Firma Novozymes A/S, Bagsvaerd, Dänemark), Chirazyme L2, Chirazyme L5, Chirazyme L8, Chirazyme L9 (alles Handelprodukte der Firma Roche Molecular Biochemicals, Mannheim, Deutschland), sowie Lipase M "Amano", Lipase F-AP 15 "Amano", Lipase AY "Amano" , Lipase N "Amano", Lipase R "Amano", Lipase A "Amano", Lipase D "Amano", Lipase G "Amano" (alles Handelsprodukte der Firma Amano, Japan).

Der Biokatalysator wird vorzugsweise in wasserfreier, bzw. teilhydratisierter Form eingesetzt. Er kann immobilisiert oder als Lyophilisat eingesetzt werden. Als Träger zur Immobilisierung können inerte organische oder anorganische Träger verwendet werden. Vorzugsweise werden als inerte Träger solche partikulären Träger verwendet bzw. sind im Enzymimmobilisat vorhanden, die eine Teilchengrößenverteilung aufweisen, bei der mindestens 90% der Teilchen eine Teilchengröße von 10 bis 5000 µm, bevorzugt von 50 µm bis 2000 µm aufweisen. Als organische Träger können insbesondere solche eingesetzt werden, die Polyacrylat, Polymethacrylat, Polyvinylstyrol, Styrol-Divinylbenzolcopolymeren, Polypropylen, Polyethylen, Polyethylenterephthalat, PTFE und/oder andere Polymere aufweisen oder aus diesen bestehen. Als Trägermaterial können, in Abhängigkeit von dem zu immobilisierenden Enzym, insbesondere saure oder basische Ionenaustauscherharze eingesetzt werden, beispielsweise Duolite A568, Duolite XAD 761, Duolite XAD 1180, Duolite XAD 7HP, Amberlite IR 120, Amberlite IR 400, Amberlite CG 50, Amberlyst 15 (alles Produkte der Fa. Rohm und Haas) oder Lewatit CNP 105 und Lewatit VP OC 1600 (Produkte der Fa. Lanxess, Leverkusen, Deutschland). Als anorganische Träger können aus dem Stand der Technik bekannte, oxidische und/oder keramische Träger eingesetzt werden. Insbesondere können als anorganische Träger beispielsweise Celite, Zeoliten, Silica, Controlled-Pore Glas (CPG) oder andere Träger, wie sie zum Beispiel in L. Cao, "Carrier-bound Immobilized Enzymes: Principles, Application and Design", Wiley-VCH: 2005, Weinheim, Deutschland, beschrieben sind, eingesetzt werden. Besonders bevorzugt bestehen die im Enzymimmobilisat vorhandenen inerten Träger bzw. die zur Herstellung der Enzymimmobilisate verwendeten inerten Träger aus Polyvinylstyrol, Polymethacrylat oder Polyacrylat.

Die Immobilisierung auf den Partikeln kann kovalent oder nichtkovalent, bevorzugt nichtkovalent erfolgen. Für die nichtkovalente Immobilisierung kann der Träger z. B. mit einer wässrigen Enzymlösung, die gegebenenfalls weitere Bestandteile, wie z. B. anorganische Salze oder Detergenzien, enthalten kann, inkubiert bzw. imprägniert werden. Diese Inkubation/Imprägnierung kann z. B. bei Temperaturen zwischen 0 °C und 50 °C, bevorzugt zwischen 0 °C und 40 °C durchgeführt werden. Vorzugsweise erfolgt die Inkubation/Imprägnierung über einen Zeitraum von wenigen Minuten bis zu einigen Stunden. Der Fortschritt der Inkubation kann durch Bestimmung der Konzentration des Enzyms in der Lösung mit den gängigen Verfahren zur Proteinbestimmung verfolgt werden. Nach Erreichen des gewünschten Immobilisierungsgrads kann der Träger vorzugsweise mit Wasser gewaschen und, wenn gewünscht, getrocknet werden.

Möglich ist auch die Verwendung ganzer Zellen, die einen geeigneten Biokatalysator enthalten, entweder als *resting cells* oder in getrockneter Form, vorzugsweise nach einer Methode des Standes der Technik permeabilisiert.

Erfindungsgemäß kann der Biokatalysator in einem Mengenverhältnis zu den eingesetzten Substraten von 0,01% (w/w) bis 300% (w/w)verwendet werden. Das bevorzugte Massenverhältnis folgt aus der spezifischen Aktivität des Biokatalysators unter den jeweiligen Reaktionsbedingungen (insbesondere in Abhängigkeit der Substrate, deren Konzentration, des Lösungsmittels und der Reaktionstemperatur). In Abhängigkeit dieser Parameter wird eine Biokatalysatormenge bevorzugt, die einen Vollumsatz der Reaktanden innerhalb eines Zeitraumes von 1 bis 96 Stunden, vorzugsweise von 8 bis 24 Stunden ermöglicht. Bevorzugt wird die Biokatalysatormenge bezogen auf die Gesamtmasse an Edukten in einem Bereich zwischen 1% (w/w) und 100% (w/w) und ganz besonders bevorzugt zwischen 1% (w/w) und 50% (w/w) insbesondere zwischen 1% (w/w) und 20% (w/w) eingesetzt.

In einer besonderen Ausführungsform wird der Biokatalysator zurückgewonnen.

Erfindungsgemäß können die Reaktanden zu Beginn der enzymatischen Umsetzung in einem molaren Verhältnis von Lysosphingolipid zu Carbonsäureester von 1:10 bis 10:1 vorliegen. Der Term "molare Verhältnis" bezieht sich hierbei auf das molare Verhältnis von Lysosphingolipid-Aminen zu Acyldonorcarboxylatgruppen. Bevorzugt werden Mengenverhältnisse zwischen 1:3 und 3:1 eingesetzt. Besonders bevorzugt werden Mengenverhältnisse zwischen 1:1,4 und 1,4:1 eingesetzt, insbesondere äquimolare Mengenverhältnisse.

Die Reaktanden können entweder im geschmolzenen Zustand ohne weitere Lösungsvermittler vorliegen oder in einem organischen Lösungsmittel gelöst oder suspendiert sein. Bevorzugt wird die Reaktion im organischen Lösungsmittel durchgeführt. Als Lösungsmittel eignen sich besonders diese, die die Reaktanden bei Temperaturen im Bereich 20°C bis 130°C in hohen Konzentrationen zu lösen vermögen. Als besonders geeignet erwiesen sich beispielsweise, ohne eine Beschränkung auf diese Lösungsmittel, Ketone wie beispielsweise Methylisobutylketon oder Cylclohexanon, sterisch gehinderte sekundäre Alkohole wie 2-Butyl-1-Oktanol, Methylcyclohexanole, 1-Methoxy-2-propanol, 2,3-Butandiol 2-Oktanol, Diacetonalkohol, 2-Methyl-2-butanol, sterisch gehinderte Ester wie 4-tert-Butylcyclohexylacetat, (2-Ethylhexyl)acetat, Cyclohexylacetat und Ether wie 1,4-Dioxan, Tetrahydrofuran und Varonic APM (Evonik Goldschmidt GmbH, Essen, Deutschland). Als ungeeignet erwiesen sich solche Lösungsmittel, insbesondere Ester, die mit signifikanter Aktivität vom Biokatalysator umgesetzt werden. Bei der Reaktion mit Phytosphingosin als Edukt und Verwendung von Buttersäureethylester konnten beispielsweise neben dem gewünschten Produkt signifikante Mengen an N-Butyrylphytosphingosin und Umesterungsprodukt mit dem eingesetzten Acyldonor (z.B. Methylstearat) nachgewiesen werden. Dasselbe gilt für die Verwendung von Essigsäurebutylester (Bildung von N-Acetylphytosphingosin und Acyldonorbutylester).

Bevorzugt wird die Reaktion unter wasserfreien Bedingungen, definiert als Wassergehalt von maximal 0,100 M, bevorzugt maximal 0,010 M und besonders bevorzugt unter maximal 0,005 M, nachgewiesen nach Karl Fischer, durchgeführt.

Hohe Substratkonzentrationen sind besonders vorteilhaft für die Reaktionsgeschwindigkeit. Bei hohen Umsätzen resultieren daraus hohe Produktkonzentrationen die sich überraschenderweise aber nicht negativ auf die Enzymaktivität bei hohen Umsätzen auswirken. Dies ist für den Fachmann insofern überraschend als dass Produktinhibition bei enzymatischen Reaktionen üblicherweise beobachtet wird. Darüber hinaus wurde überraschenderweise gefunden, dass die Entfernung eines der Reaktiönsprodukte (Ceramid oder Alkohol) aus der Reaktionsmischung nicht zwingend zur Erreichung sehr hoher bis quantitativer Umsätze notwendig ist. Nach dem, dem Fachmann geläufigen, Prinzip von Le Chatelier wäre dies bei der vorliegenden Gleichgewichtsreaktion zu erwarten gewesen. Darüber ließen die Reaktionsumsätze des Standes der Technik (WO94/26919, Gist-Brocades) von lediglich 67% bis 78% diese Notwendigkeit erwarten. Erfindungsgemäß können allerdings auch niedrige Substratkonzentrationen effizient umgesetzt werden.

Bevorzugt im Sinne der Erfindung ist eine Reaktandenkonzentration zu Beginn der Reaktion im Bereich zwischen 0,01 M und 3 M, insbesondere im Bereich zwischen 0,2 M und 2 M und besonders bevorzugt im Bereich zwischen 0,5 M und 1,0 M je Reaktand liegt.

Bezüglich der Reaktionstemperatur wurde gefunden, dass insbesondere solche Temperaturen geeignet sind bei denen die Substrate homogen im Lösungsmittel löslich sind. Vorteilhaft erwiesen sich dabei Reaktionstemperaturen im Bereich zwischen 20°C und 130°C. Insbesondere bei hohen Temperaturen ist aufgrund von Farbveränderungen auf eine sorgfältige Entgasung der Reaktionsmischung zu achten. Überaschenderweise wurde allerdings gefunden, dass Vorkehrungen solcherart bei Reaktionstemperaturen von 100°C und darunter nicht notwendig sind. Bevorzugt sind daher Reaktionstemperaturen im Bereich von 40 °C bis 90°C und ganz besonders bevorzugt der Bereich zwischen 70 °C und 85 °C.

Die Reaktion wird bevorzugt unter einem Druck von kleiner 1 bar, bevorzugt kleiner 0,5 bar und besonders bevorzugt kleiner 0,05 bar durchgeführt.

Die Reaktionsführung kann im batch-Verfahren (Rührkessel) oder im Festbettreaktor kontinuierlich oder semi-kontinuierlich durchgeführt werden. Insbesondere beim batch-Verfahren war von einer hohen Inaktivierungsrate des Biokatalysators auszugehen. Zu Nennen ist hier die mechanische Zerstörung des Biokatalysators aufgrund des auftretenden mechanischen Stresses durch Rühren, sowie thermische Inaktivierung des Biokatalysators gepaart mit inaktivierenden Effekten hervorgerufen durch Reaktanden und Lösungsmittel. Solche Effekte sind dem Fachmann bekannt und adäquate Abhilfen sind literaturbekannt. So kann beispielsweise bei auftretender mechanischer Abnutzung des Biokatalysators im Rührverfahren diese durch Einsatz eines Festbetts umgangen werden. Weitere Reaktortypen wie sie dem Fachmann bekannt sind eignen sich ebenfalls zur Verhinderung von mechanischer Enzyminaktivierung.

Weiterhin ist bei der Verwendung von Säureestern als Acyldonoren mit chemischer Inaktivierung des Biokatalysators durch die freiwerdenden Alkohole zu rechnen (z.B.: Y. Yoshida et al., J. Biosci. Bioeng., 2006, 102(1), 66-68). Methoden zur Umgehung solcher Inaktivierungen sind dem Fachmann bekannt und umfassen beispielsweise die destillative Abtrennung des Alkohols aus dem Reaktionsgemisch oder die Anwendung von Membranverfahren. Überraschenderweise wurde allerdings gefunden, dass auch ohne Anwendung der oben beschriebenen Verfahren der Biokatalysator mehrfach und ohne signifikanten Aktivitätsverlust wiederverwendet werden kann.

Insbesondere war zu erwarten, dass bei Verwendung natürlicher Glyceride, wenigstens intermediaer Di- und Monoglyceride in der Reaktionsmischung akkumulieren und bei nichtvollständigem Umsatz zu erheblichen Verunreinigungen des Produktes führen. Darüber hinaus war aufgrund des tensidischen Charakters der oben beschriebenen Partialglyceride zu erwarten, dass dies zu erheblicher Inaktivierung des Biokatalysators führen könne. Überaschenderweise wurde allerdings gefunden, dass die zu erwartenden Partialglyceride praktisch nicht nachweisbar waren. Allenfalls Spuren (<1% w/w) konnten im Reaktionsverlauf durch gaschromatographische Analyse festgestellt werden.

Allen im Stand der Technik beschriebenen Verfahren ist gemein, dass die erhaltenen Produkte Zusammensetzungen aus Sphingolipid und hohen Anteilen (bis zu 19% des Gesamtproduktes) des korrespondierenden N,O-Diacylierungsproduktes sind. Diese Zusammensetzungen sind allesamt in beispielsweise kosmetischen Komposititionen nicht einsetzbar, da der Anteil an N,O-Diacylierungsprodukt nicht kleiner 5 % ist. Überaschenderweise wurde weiterhin gefunden, dass in dem erfindungsgemäßen Verfahren die Amidierung der sekundären Aminfunktion gegenüber der Veresterung einer der Alkoholfunktionen deutlich bevorzugt ist. Ein gegenteiliges Verhalten insbesondere eine Bevorzugung der primären, also sterisch leicht zugänglichen Alkoholfunktion wäre zu erwarten gewesen. Ähnliches ist im Stand der Technik (WO94/26919, Gist-Brocades) beschrieben; die dort erhaltenen Reaktionsprodukte enthielten bei Verwendung einer bakteriellen Lipase und bereits bei einem molaren Überschuss von 60% des Acyldonors über Phytosphingosin, signifikante Anteile an N, O-Diacylierungsprodukt (Mono- zu Diacylierungsprodukt > 1:5).

In dem erfindungsgemäßen Verfahren konnte hingegen gezeigt werden, dass sogar bei einem 3,6-fachen Überschuss an Acyldonor über Lysosphingolipid keine signifikante Diacylierung eintritt solange noch Lysosphingolipid im Reaktionsansatz vorhanden ist. Erst ab vollständigem Umsatz von Lysosphingolipid zum Sphingolipid konnte langsame Veresterungsaktivität nachgewiesen werden. Bei stöchiometrischer Verwendung von Lysosphingolipid und Acyldonor wurde die oben beschriebene unerwünschte Nebenreaktion kaum beobachtet.

Offenbart wird ein Verfahren, mit dem besonders reine N-Acylphytosphingosine bereits als Rohprodukte aus der enzymatischen Umsetzung erhalten werden. Vorzugsweise liegt der Umsatz in Bezug auf die Zielverbindung gemäß Formel I bei über 80 %, besonders bevorzugt bei über 85% und ganz besonders bevorzugt bei mehr als 90%% des theoretisch zu erwartenden Umsatzes.

Das beschriebene Verfahren eignet sich weiterhin zur Herstellung einer Zusammensetzung enthaltend Formel I und von 0,001 bis 19 Massen-%, bevorzugt 0,005 bis 19 Massen-% besonders bevorzugt 0,01 bis 5 Massen-% des korrespondierenden N,O-Diacylierungsproduktes.

Somit sind Zusammensetzungen enthaltend Formel I und von 0,001 bis 19 Massen-%, bevorzugt 0,005 bis 19 Massen-% besonders bevorzugt 0,01 bis 5 Massen-% des korrespondierenden N,O-Diacylierungsproduktes ebenfalls offenbart.

Weiterhin offenbart ist eine Methode zur Isolierung des Sphingolipids aus der Reaktionsmischung. Aufgrund seiner vorteilhaften physikalischen Eigenschaften kann das Sphingolipid leicht durch Kristallisation / Präzipitation aus der Reaktionslösung entfernt werden und somit die bereits hohe Reinheit des Rohproduktes auf einfache Weise noch gesteigert werden.

Beschriebene Sphingolipide und Zusammensetzungen sind besonders geeignet für die Herstellung von kosmetischen und pharmazeutischen Formulierungen. Daher sind kosmetische oder pharmazeutische Formulierungen enthaltend mindestens ein erfindungsgemäßes Sphingolipid und/oder eine beschriebene Zusammensetzung ebenfalls offenbart.

In den nachfolgend aufgeführten Beispielen wird die vorliegende Erfindung beispielhaft beschrieben.

### Beispiele:

### Beispiel 1: Tristearin als Acyldonor

Es wurden enzymatische Umsetzungen unter Verwendung von Glcerintristearat (Tristearin) als Acyldonor durchgeführt. Zu einer Lösung von 5,4 g Phytosphingosin und 5 g Tristearin in 17g Dioxan bei 80°C wurden 0,5 g Novozyme 435 zugegeben und bei Normaldruck gerührt. Es wurden periodisch Proben entnommen und gaschromatographisch analysiert.

Dieser Versuch steht stellvertretend fuer die Verwendung weiterer Glyceride bei dem qualitativ derselbe Reaktionsverlauf, insbesondere die Nicht-Nachweisbarkeit intermediaerer Partialglyceride, beobachtet wird.

Ein vollständiger Umsatz des Acyldonors ist möglich. Überraschenderweise ergab die gaschromatographische Analyse dass intermediär keine Partialglyceride (Glycerindistearat oder Glycerinmonostearat) in signifikanten Mengen beobachtbar waren.

### Beispiel 2: Enzymatische Umsetzung von Phytossphingosin mit natürlichen Ölen

5 g Phytosphingosin werden jeweils in 8,5 g 2-Methyl-2-butanol gelöst und mit einem Äquivalent (äquimolares Verhältnis von Phytosphingosin zu Carboxylatmenge) des natürlichen Öles versetzt. Die spezifische Einwage des Öles ergibt sich aus der Verseifungszahl des Öles; die daraus errechnete Carboxylatmenge dient zur Berechnung der Ansatzmenge. Die Reaktionsmischung wird nach Zugabe von jeweils 0,75 g Novozym 435 für 24h bei 80°C unter N₂-Atmosphäre gerührt. Anschließend wird die Reaktionsmischung heiß filtriert und das Lösungsmittel *in vacuo* entfernt. Das erhaltene Produkt wird gaschromatographisch analysiert; außerdem werden Jodzahl und Farbzahl (nach Hazen) bestimmt. In keinem der Produkte waren Partialglyceride > 1% w/w nachweisbar.

| Öl | Umsatz [%]^{a} | Farbzahl ^{b} | Jodzahl^{c} | Peroxidzahl^{d} |
|---|---|---|---|---|
| Leinsamenöl ^{e} | 98,7 | 21,1 | 95 | 4 |
| Leinsamenöl ^{f} | 99,2 | 6, 9 | 96 | 2 |
| Leinsamenöl ^{g} | 96, 6 | 12,7 | 96 | 5 |
| Olivenöl | 98,1 | 8,2 | 41 | **4** |
| Rapsöl | 99,1 | 5,3 | 52 | 2 |
| Sonnenblumenöl | 99,3 | 4,6 | 63 | 2 |
| Fischöl (Menhaden) | 99,8 | 6,3 | 74 | **6** |
| Fischöl (Lebertran) | 99,4 | 6,0 | 67 | 4 |

| | | | | |
|---|---|---|---|---|
| a: bezogen auf Phytosphingosin, laut gaschromatographischer Analyse b: Farbzahl nach Hess-Ives, als 1M Lösung in 2-Me-2-Butanol c: Jodzahl nach Wijs, als 1M Lösung in 2-Me-2-Butanol d: Peroxidzahl in mEq O₂/kg e: Reaktion unter Normalatmosphäre f: Reaktion unter Stickstoffatmosphäre g: Reaktion bei 50°C unter Normalatmosphäre | | | | |

### Beispiel 3: Verwendung von Sphingosin

3 g Sphingosin wird in 5 g 2-Methyl-2-butanol gelöst und mit einem Äquivalent Leinöl (Carboxylatmenge über Verseifungszahl bestimmt) versetzt. Nach Zugabe von 0,5 g Novozym 435 wird die Reaktionsmischungen 24h bei 80°C unter Stickstoffatmosphaere gerührt. Anschließend wird heiß filtriert und das Lösungsmittel unter vermindertem Druck entfernt. Das so erhaltene Produkt wird gaschromatographisch analysiert. Es wird ein Umsatz von 98,0% ohne nachweisbare Spuren von Mono- bzw. Diglyceriden erreicht.

### Beispiel 4: Verwendung von Sphinganin

3 g Sphinganin werden in 5 g 2-Methyl-2-butanol gelöst und mit einem Äquivalent Leinöl (Carboxylatmenge über Verseifungszahl bestimmt) versetzt. Nach Zugabe von 0,5 g Novozym 435 wird die Reaktionsmischung 24h bei 80°C unter Stickstoffatmosphaere gerührt. Anschließend wird heiß filtriert und das Lösungsmittel unter vermindertem Druck entfernt. Das so erhaltene Produkt wird gaschromatographisch analysiert. Der Umsatz beträgt 99,7% ohne nachweisbare Spüren von Mono- bzw. Diglyceriden.

## Patentansprüche

1. Verfahren zur biokatalytischen Herstellung von Sphingolipiden der allgemeinen Formel I 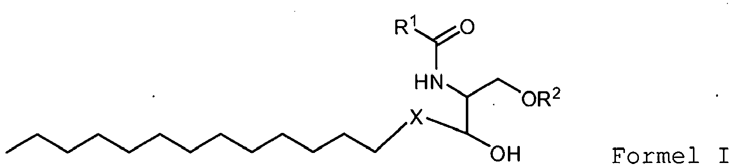 durch Umsetzung eines Lysosphingolipids der allgemeinen Formel II 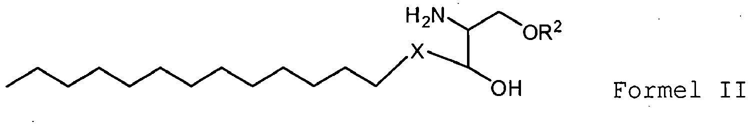 mit mindestens einem Carbonsäureester ausgewählt aus der Gruppe umfassend Verbindungen der allgemeinen Formeln IIIa-e 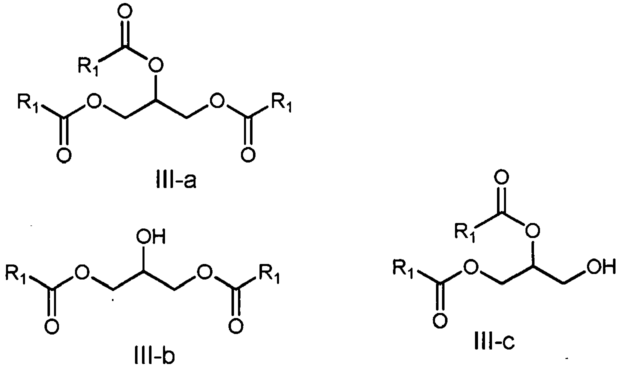 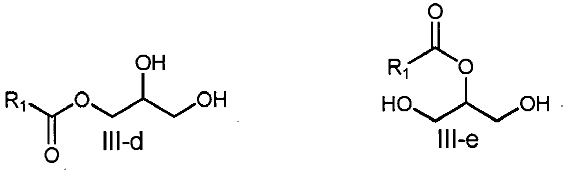 in Gegenwart eines Biokatalysators, der mindestens eine Carboxylester-Hydrolase der Enzymklasse E.C. 3.1.1 umfasst,
wobei R¹ unabhängig voneinander und gegebenenfalls unterschiedliche, lineare oder verzweigte, gegebenenfalls eine oder mehrere Mehrfachbindungen und/oder aromatische oder heteroaromatische Ringe enthaltende, gegebenenfalls durch Sauerstoffatome oder Ester- oder Amidfunktionalitäten unterbrochene, gegebenenfalls durch mindestens eine weitere Gruppe ausgewählt aus Alkyl- Hydroxy-, Keto- oder Amingruppen substituierte Alkylketten mit 2 bis 55 Kohlenstoffatomen darstellt,
R² H, Phosphocholin, Ethanolamin, Serin oder einen Zu-cker darstellt,
X CH=CH, CH₂-CH₂ oder CH₂-HCOH darstellt.

2. Verfahren nach Anspruche 1, **dadurch gekennzeichnet, dass** der Carbonsäureester der Ester mindestens einer Carbonsäure mit Glycerin, bei dem die Carbonsäure ausgewählt ist aus der Gruppe der natürlich vorkommenden Fettsäuren auf Basis natürlicher pflanzlicher oder tierischer Öle mit 6-30 Kohlenstoffatomen ist.

3. Verfahren nach einem der Ansprüche 1 oder 2, **dadurch gekennzeichnet, dass** vor der enzymatischen Umsetzung eine Herstellung des Lysosphingolipids durch Deprotonierung eines Säureadditionsprodukts des Lysosphingolipids steht.

4. Verfahren nach Anspruch 3, **dadurch gekennzeichnet, dass** zwischen der Deprotonierung des Säureadditionsprodukts des Lysosphingolipids und biokatalytischer Herstellung ein Filtrationsschritt erfolgt.

5. Verfahren nach mindestens einem der Ansprüche 3 oder 4, **dadurch gekennzeichnet, dass** als Säureadditionsprodukt des Lysosphingolipids das Carbonsäurecarboxylat, Sulfat, Phosphat, Nitrat, Carbonat, Hydroxid oder Halogenid des Lysosphingolipids eingesetzt wird.

6. Verfahren nach mindestens einem der Ansprüche 3 bis 5, **dadurch gekennzeichnet, dass** zur Deprotonierung eine organische oder anorganische Base eingesetzt wird.

7. Verfahren nach Anspruch 6, **dadurch gekennzeichnet, dass** Basen eingesetzt werden, die nach Protonierung kein Wasser frei setzen.

8. Verfahren nach mindestens einem der Ansprüche 6 oder 7, **dadurch gekennzeichnet, dass** als Base ein Alkalimetallalkoholat eingesetzt wird.

9. Verfahren nach mindestens einem der Ansprüche 6 bis 8, **dadurch gekennzeichnet, dass** das molare Verhältnis zwischen Säureadditionsprodukt des Lysosphingolipids und Base im Bereich zwischen 10 zu 1 bis 0,05 zu 1.

10. Verfahren nach einem der Ansprüche 1 bis 9, **dadurch gekennzeichnet, dass** der Gehalt an Partialglyceriden kleiner als 2,5% w/w bezogen auf die Masse der Einwaage des Carbonsäureesters der allgemeinen Formeln IIIa-e als Edukt ist.

11. Verfahren nach mindestens einem der Ansprüche 1 bis 10, **dadurch gekennzeichnet, dass** die Carboxylester-Hydrolase sich isolieren lässt aus einem Organismus ausgewählt aus der Gruppe der Gattungen *Aspergillus*, *Bipolaris, Candida, Fusarium, Geotrichum, Humicola, Microsporum, Mucor, Pichia, Penicillium, Rhizopus, Rhizomucor, Nocardia, Saccharomyces, Streptomyces, Thermomyces, Trichosporon, Zygosaccharomyces.*

12. Verfahren nach mindestens einem der Ansprüche 1 bis 11, **dadurch gekennzeichnet, dass** die Biokatalysatormenge bezogen auf die Gesamtmasse an Edukten in einem Bereich zwischen 1% (w/w) und 100% (w/w) eingesetzt wird.

13. Verfahren nach mindestens einem der Ansprüche 1 bis 12, **dadurch gekennzeichnet, dass** der Biokatalysator zurückgewonnen wird.

14. Verfahren nach mindestens einem der Ansprüche 1 bis 13, **dadurch gekennzeichnet, dass** die Reaktanden zu Beginn der enzymatischen Umsetzung in einem molaren Verhältnis von Lysosphingolipid zu Carbonsäureester von 1:10 bis 10:1 vorliegen

15. Verfahren nach mindestens einem der Ansprüche 1 bis 14, **dadurch gekennzeichnet, dass** die Reaktion im organischen Lösungsmittel durchgeführt wird.

16. Verfahren nach mindestens einem der Ansprüche 1 bis 15, **dadurch gekennzeichnet, dass** die Reaktion unter wasserfreien Bedingungen, definiert als Wassergehalt von maximal 0,100 M, nachgewiesen nach Karl Fischer, durchgeführt wird

17. Verfahren nach mindestens einem der Ansprüche 1 bis 16, **dadurch gekennzeichnet, dass** die Reaktandenkonzentration zu Beginn der Reaktion im Bereich zwischen 0,01 M bis 3 M je Reaktand liegt.

18. Verfahren nach mindestens einem der Ansprüche 1 bis 17, **dadurch gekennzeichnet, dass** die Reaktionstemperatur im Bereich zwischen 20 °C und 130 °C liegt.

19. Verfahren nach mindestens einem der Ansprüche 1 bis 18, **dadurch gekennzeichnet, dass** die Reaktion unter einem Druck von kleiner 1 bar durchgeführt wird.

## Claims

1. Process for the biocatalytic preparation of sphingolipids of the general formula I 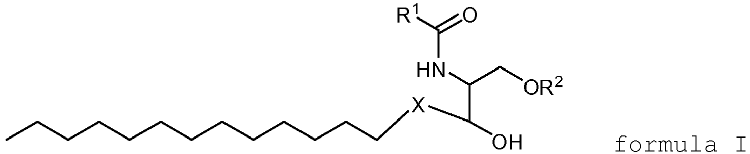 by reacting a lysosphingolipid of the general formula II 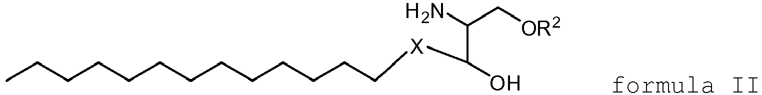 with at least one carboxylic ester selected from the group comprising compounds of the general formulae IIIa-e 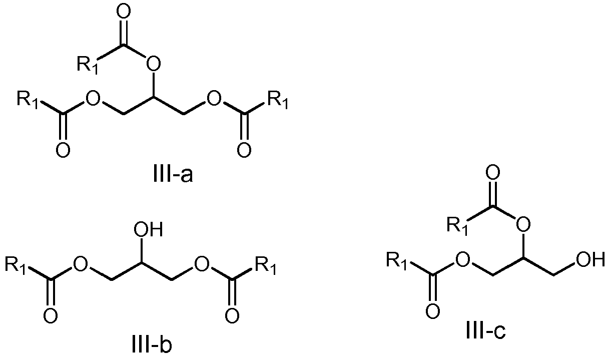 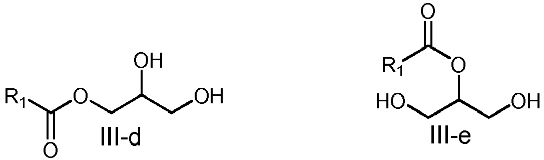 in the presence of a biocatalyst which includes at least one carboxylic ester hydrolase of enzyme class E.C. 3.1.1,
where R¹ represents independently of one another and optionally different, linear or branched, alkyl chains which have 2 to 55 carbon atoms, optionally comprise one or more multiple bonds and/or aromatic or heteroaromatic rings, are optionally interrupted by oxygen atoms or ester or amide functionalities and are optionally substituted by at least one further group selected from alkyl, hydroxy, keto or amine groups,
R² represents H, phosphocholine, ethanolamine, serine or a sugar,
X represents CH=CH, CH₂-CH₂ or CH₂-HCOH.

2. Process according to Claim 1, **characterized in that** the carboxylic ester is the ester of at least one carboxylic acid with glycerol, where the carboxylic acid is selected from the group of naturally occurring fatty acids based on natural plant or animal oils with 6-30 carbon atoms.

3. Process according to either of Claims 1 or 2, **characterized in that** the lysosphingolipid is prepared by deprotonation of an acid addition product of the lysosphingolipid before the enzymatic reaction.

4. Process according to Claim 3, **characterized in that** a filtration step takes place between the deprotonation of the acid addition product of the lysosphingolipid and biocatalytic preparation.

5. Process according to at least one of Claims 3 or 4, **characterized in that** the carboxylic acid carboxylate, sulphate, phosphate, nitrate, carbonate, hydroxide or halide of the lysosphingolipid is employed as acid addition product of the lysosphingolipid.

6. Process according to at least one of Claims 3 to 5, **characterized in that** an organic or inorganic base is employed for the deprotonation.

7. Process according to Claim 6, **characterized in that** bases which do not liberate any water after protonation are employed.

8. Process according to at least one of Claims 6 or 7, **characterized in that** an alkali metal alcoholate is employed as base.

9. Process according to at least one of Claims 6 to 8, **characterized in that** the molar ratio between acid addition product of the lysosphingolipid and base is in the range between 10:1 to 0.05:1.

10. Process according to any of Claims 1 to 9, **characterized in that** the content of partial glycerides is less than 2.5% w/w based on the mass of the initial weight of the carboxylic ester of the general formulae IIIa-e as precursor.

11. Process according to at least one of Claims 1 to 10, **characterized in that** the carboxylic ester hydrolase can be isolated from an organism selected from the group of genera *Aspergillus, Bipolaris, Candida, Fusarium, Geotrichum, Humicola, Microsporum, Mucor, Pichia, Penicillium, Rhizopus, Rhizomucor, Nocardia, Saccharomyces, Streptomyces, Thermomyces, Trichosporon, Zygosaccharomyces.*

12. Process according to at least one of Claims 1 to 11, **characterized in that** the amount of biocatalyst, based on the total mass of precursors, is employed in a range between 1% (w/w) and 100% (w/w).

13. Process according to at least one of Claims 1 to 12, **characterized in that** the biocatalyst is recovered.

14. Process according to at least one of Claims 1 to 13, **characterized in that** the reactants are present at the start of the enzymatic reaction in a molar ratio of lysosphingolipid to carboxylic ester of from 1:10 to 10:1.

15. Process according to at least one of Claims 1 to 14, **characterized in that** the reaction is carried out in the organic solvent.

16. Process according to at least one of Claims 1 to 15, **characterized in that** the reaction is carried out under anhydrous conditions, defined as a water content not exceeding 0.100 M detected by the Karl Fischer method.

17. Process according to at least one of Claims 1 to 16, **characterized in that** the reactant concentration at the start of the reaction is in the range between 0.01 M to 3 M for each reactant.

18. Process according to at least one of Claims 1 to 17, **characterized in that** the reaction temperature is in the range between 20°C and 130°C.

19. Process according to at least one of Claims 1 to 18, **characterized in that** the reaction is carried out under a pressure of less than 1 bar.

## Revendications

1. Procédé de fabrication biocatalytique de sphingolipides de formule générale I 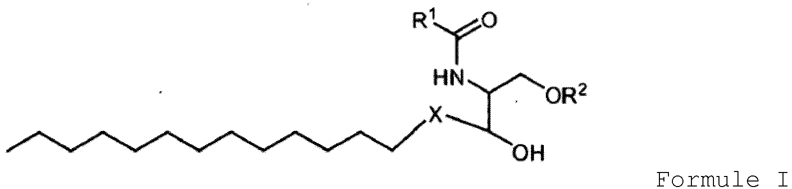 par mise en réaction d'un lysosphingolipide de formule générale II 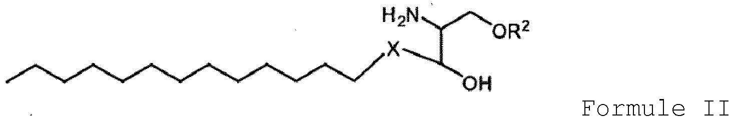 avec au moins un ester d'acide carboxylique choisi dans le groupe comprenant les composés de formule générale IIIa à e 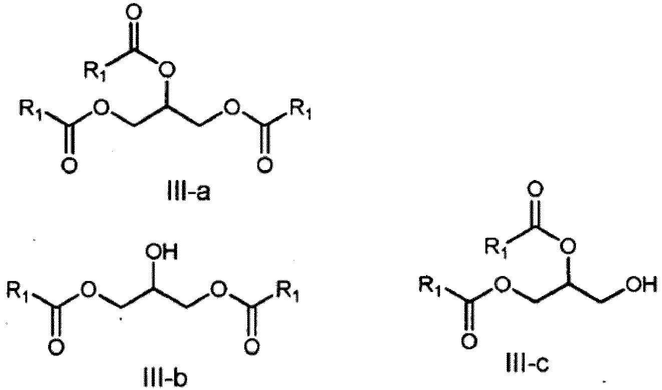 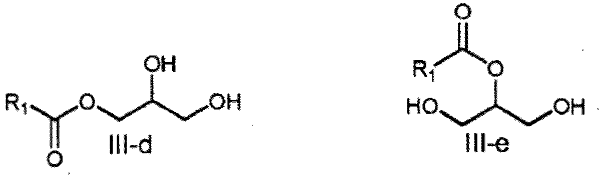 en présence d'un biocatalyseur qui comprend au moins une hydrolase d'ester carboxylique de la classe enzymatique E.C. 3.1.1,
les R¹ représentant indépendamment les uns des autres des chaînes alkyle de 2 à 55 atomes de carbone, éventuellement différentes, linéaires ou ramifiées, éventuellement contenant une ou plusieurs liaisons multiples et/ou un ou plusieurs cycles aromatiques ou hétéroaromatiques, éventuellement interrompues par des atomes d'oxygène ou des fonctionnalités ester ou amide, éventuellement substituées par au moins un groupe supplémentaire choisi parmi les groupes alkyle, hydroxy, céto ou amine,
R² représentant H, phosphocholine, éthanolamine, sérine ou un sucre,
X représentant CH=CH, CH₂-CH₂ ou CH₂-HCOH.

2. Procédé selon la revendication 1, **caractérisé en ce que** l'ester d'acide carboxylique est l'ester d'au moins un acide carboxylique avec de la glycérine, l'acide carboxylique étant choisi dans le groupe des acides gras naturels à base d'huiles végétales ou animales naturelles de 6 à 30 atomes de carbone.

3. Procédé selon l'une quelconque des revendications 1 ou 2, caractérisé en qu'une préparation du lysosphingolipide par déprotonation d'un produit d'addition acide du lysosphingolipide a lieu avant la réaction enzymatique.

4. Procédé selon la revendication 3, **caractérisé en ce qu'**une étape de filtration a lieu entre la déprotonation du produit d'addition acide du lysosphingolipide et la fabrication biocatalytique.

5. Procédé selon au moins l'une quelconque des revendications 3 ou 4, **caractérisé en ce que** le carboxylate d'acide carboxylique, le sulfate, le phosphate, le nitrate, le carbonate, l'hydroxyde ou l'halogénure du lysosphingolipide est utilisé en tant que produit d'addition acide du lysosphingolipide.

6. Procédé selon au moins l'une quelconque des revendications 3 à 5, **caractérisé en ce qu'**une base organique ou inorganique est utilisée pour la déprotonation.

7. Procédé selon la revendication 6, **caractérisé en ce que** des bases qui ne libèrent pas d'eau après la protonation sont utilisées.

8. Procédé selon au moins l'une quelconque des revendications 6 ou 7, **caractérisé en ce qu'**un alcoolate de métal alcalin est utilisé en tant que base.

9. Procédé selon au moins l'une quelconque des revendications 6 à 8, **caractérisé en ce que** le rapport molaire entre le produit d'addition acide du lysosphingolipide et la base se situe dans la plage comprise entre 10 sur 1 et 0,05 sur 1.

10. Procédé selon l'une quelconque des revendications 1 à 9, **caractérisé en ce que** la teneur en glycérides partiels est inférieure à 2,5 % m/m par rapport à la masse de l'ester d'acide carboxylique des formules générales IIIa à e en tant que réactif.

11. Procédé selon au moins l'une quelconque des revendications 1 à 10, **caractérisé en ce que** l'hydrolase d'ester carboxylique peut être isolée à partir d'un organisme choisi dans le groupe constitué par les genres *Aspergillus, Bipolaris, Candida, Fusarium, Geotrichum, Humicola, Microsporum, Mucor, Pichia, Penicillium, Rhizopus, Rhizomucor, Nocardia, Saccharomyces, Streptomyces, Thermomyces, Trichosporon, Zygosaccharomyces.*

12. Procédé selon au moins l'une quelconque des revendications 1 à 11, **caractérisé en ce que** la quantité de biocatalyseur utilisée par rapport à la masse totale de réactifs se situe dans une plage comprise entre 1 % (m/m) et 100 % (m/m).

13. Procédé selon au moins l'une quelconque des revendications 1 à 12, **caractérisé en ce que** le biocatalyseur est récupéré.

14. Procédé selon au moins l'une quelconque des revendications 1 à 13, **caractérisé en ce que** les réactifs sont présents au début de la réaction enzymatique en un rapport molaire entre le lysosphingolipide et l'ester d'acide carboxylique de 1:10 à 10:1.

15. Procédé selon au moins l'une quelconque des revendications 1 à 14, **caractérisé en ce que** la réaction est réalisée dans un solvant organique.

16. Procédé selon au moins l'une quelconque des revendications 1 à 15, **caractérisé en ce que** la réaction est réalisée dans des conditions anhydres, définies comme une teneur en eau d'au plus 0,100 M, détectée selon Karl Fischer.

17. Procédé selon au moins l'une quelconque des revendications 1 à 16, **caractérisé en ce que** la concentration en réactifs au début de la réaction se situe dans la plage comprise entre 0,01 M et 3 M par réactif.

18. Procédé selon au moins l'une quelconque des revendications 1 à 17, **caractérisé en ce que** la température de réaction se situe dans la plage comprise entre 20 °C et 130 °C.

19. Procédé selon au moins l'une quelconque des revendications 1 à 18, **caractérisé en ce que** la réaction est réalisée sous une pression de moins de 1 bar.
